# EUROPEAN PATENT SPECIFICATION

(11) **EP 2 252 956 B1**
(45) Date of publication and mention of the grant of the patent: **26.12.2018**
(21) Application number: 09717353.8
(22) Date of filing: 02.03.2009
(51) Int. Cl.: G06F 19/00, A61M 5/00, G06Q 50/22, A61M 5/142

(54) **INSULIN PUMP WITH REPLACEMENT CAPABILITIES**
AUSTAUSCHBARE INSULINPUMPE
POMPE À INSULINE

(30) Priority: 03.03.2008 EP 08405064
(43) Date of publication of application: 24.11.2010
(73) Proprietor: Roche Diabetes Care GmbH, 68305 Mannheim (DE); F. Hoffmann-La Roche AG, 4070 Basel (CH)
(72) Inventor: REMDE, Axel, CH-3432 Lützelflüh-Goldbach (CH)
(74) Representative: Rentsch Partner AG
(86) International application number: PCT/EP2009/001458
(87) International publication number: WO 2009/109344

(56) References cited:
- WO-A-2006/097453
- US-A1- 2007 124 002

## Description

### Technical field

The invention relates to an insulin delivery system comprising a first insulin pump and a second insulin pump, allowing replacement of the first insulin pump by the second insulin pump via autonomous program transfer. The invention further relates to a method for replacing a first insulin pump by a second insulin pump and to an insulin pump which may be used in the insulin delivery system and may be used in the method according to the invention.

### Prior art

Remotely controlled insulin pumps are used for the continuous subcutaneous infusion of insulin to patients with diabetes. The insulin, which is present in a reservoir of the insulin pump, is conveyed into the patient's body according to a patient- and time-of-day dependent basal delivery schedule via a subcutaneous cannula. In addition to this basal delivery, bolus insulin delivery may be performed out of the same reservoir on demand, for example for compensating carbohydrate intake and for correcting undesirably raised blood glucose values. Some modern insulin pumps have programmable remote controllers. Those remote controllers are typically of a design which is similar to a cell phone or a PDA. Alternatively, a standard device such as a cell phone or a PDA may be used as remote controller. For those insulin pumps, the user interface of the insulin pump itself may be very limited or may even be missing. This is especially favourable with respect to robustness and water tightness as well as for cost reasons.

Insulin pumps which are designed to be fully remote controlled are disposable pumps which are designed to be used for a number of days and to be disposed afterwards. When such a pump is replaced, an infusion program including all required control data, such as the basal delivery schedule, the date and the time of day are transferred onto the new pump from the remote controller. Prior to this transfer, the memory of the insulin pump comprises the control firmware but no patient-specific infusion program.

Some insulin pumps, and especially insulin pumps of the disposable type, are designed as patch pumps which are carried by the patient directly at the infusion site and are fixed to the skin by an adhesive layer. Those pumps comprise a cannula and an automatic inserter which automatically inserts the cannula into to patient's subcutaneous tissue from an initially retracted position inside the housing. Those pumps are provided with a readily built-in reservoir which is filled by the patient prior to use and readily built-in battery. After a using time of some days, the pump is disposed as a whole.

For fully remote controlled insulin pumps, the remote controller is required for all user operations such as programming the delivery of an insulin bolus or replacing the pump by a new one. However, in some situations a remote controller may not be available. This is the case, for example, if the remote controller is defective or lost or forgotten, e.g., when travelling. According to the state of the art, the insulin pump therapy has to be interrupted as a whole in such a situation.

The patent application WO2007/056592 discloses an infusion pump which can communicate with another infusion pump. User information or treatment parameters can be transferred from the one pump to the other pump. The document does not disclose the initiation and the process of such a transfer.

### Disclosure of the invention

It is the overall object of the invention to provide insulin pumps and insulin delivery systems that allows a diabetic patient to continue the insulin pump therapy in an easy and fool proof way if the insulin pump has to be replaced. The insulin pumps may be of any general type known in the art. For exemplary purposes, disposable patch pumps as described above is assumed in the following since the invention is especially favourable in this context. However, the insulin pumps may also be of another type known in the art, as disclosed, for example in the application WO2000010628A1.

According to one aspect of the invention, the object is achieved by providing an insulin delivery system which comprises a first insulin pump and a second insulin pump. Each of the first insulin pump and the second insulin pump comprises:
a) a memory which is configured to store an infusion program,
b) a control unit which is configured to control the operation of the insulin pump,
c) a communication interface for data exchange with the other of the first insulin pump and the second insulin pump.
The control unit of the first insulin pump is configured to autonomously perform the steps of
d) monitoring, during operation of the first insulin pump according to the infusion program, a status of the first insulin pump, wherein the status comprises at least one of a filling state of an insulin reservoir of the insulin pump, the status of a battery of the insulin pump, and an error status of the insulin pump,
e) activating a search for the presence of the second insulin pump when a predetermined status is assumed by the first insulin pump, and, upon detection of the presence of the second insulin pump,
f) transmitting, via the communication interface of the first insulin pump, the infusion program stored in the memory of the first insulin pump to the second insulin pump.
The control unit of the second insulin pump is configured to perform the steps of
g) searching for the presence of the first insulin pump, and, upon detection of the presence of the first insulin pump,
h) receiving, via the communication interface of the second insulin pump, an infusion program from the first insulin pump, and
i) storing the infusion program in the memory of the second insulin pump.

The term 'infusion program' is used in the sense of data and parameters necessary for the insulin pump to operate as intended. It comprises in particular a patient-specific basal delivery schedule as well as the current time of day and optionally the current date. An operation mode in which the insulin pump performs delivery according the infusion program is referred to as 'delivery mode'.

For continuing the therapy, the infusion program is of major importance since it is the basis for an appropriate insulin infusion according to the patient's individual need. In contrast to the basal administration, the additional insulin boli may also be administered whenever required by alternative means, such as an insulin pen or a syringe. Therefore, an insulin infusion system according to the invention allows the patient to continue the therapy even if no remote controller is present and/or if the insulin pumps have no user interface for initiating or modifying the drug administration.

In an insulin infusion system according to the invention, the pump replacement is simple and fail-proof since the detection of the presence of the second insulin pump and the subsequent transfer of the infusion program are carried out autonomously, that is, without the need for user interactions.

The first insulin pump and the second insulin pump of the system may be designed or configured such that the first insulin pump only has the capability of transmitting the infusion program to the second insulin pump and the second insulin pump only has the capability of receiving an infusion program from the first insulin pump. Alternatively, either of the two insulin pumps may both transmit and receive an infusion program. In particular embodiments, however, the first insulin pump and the second insulin pump are identical insulin pumps, each being generally capable of acting as first insulin pump or as second insulin pump. Both insulin pumps may especially be disposable insulin pumps which are typically provided in a kit of, for example ten individual pumps. Alternatively, the first insulin pump may, for example, be the generally used insulin pump while the second insulin pump is an additional backup pump.

According to another aspect, the present disclosure provides a method for replacing a first insulin pump by a second insulin pump. The method comprises the steps of:
a) operating the first insulin pump according to an infusion program stored in a memory of the first insulin pump, and autonomously performing the steps of:
b) activating a search for the presence of the second insulin pump, and, upon detection of the presence of the second insulin pump,
c) transmitting, via communication interfaces of the first insulin pump and the second insulin pump, the infusion program from the first insulin pump to the second insulin pump.

The first insulin pump and the second insulin pump may especially belong to an insulin infusion system as defined above.

The method may comprise the step of monitoring, during operation of the first insulin pump according the infusion program, a status of the first insulin pump and activating the search for the presence of the second insulin pump when a predetermined status is assumed by the first insulin pump.

For this type of embodiment, the search for the presence of the second insulin pump is carried out if the status of the first insulin pump indicates that it should be replaced by the second insulin pump.

According to a still further aspect of the invention, the object is achieved by providing insulin pumps. An insulin pump according to this aspect of the invention comprises:
a) a communication interface for data exchange with a replacement pump,
b) a memory which is configured to store an infusion program, and
c) a control unit which is configured to control operation of the insulin pump and which is further configured to transmit, upon detecting the presence of a replacement pump, via the communication interface, the infusion program stored in the memory to the replacement pump.
The control unit is configured to autonomously perform the steps of
d) monitoring, during operation of the insulin pump according to the infusion program, a status of the insulin pump, wherein the status comprises at least one of a filling state of an insulin reservoir of the insulin pump, the status of a battery of the insulin pump, and an error status of the insulin pump,
e) activating a search for the presence of a replacement pump when a predetermined status is assumed by the insulin pump, and
f) transmitting, upon detecting the presence of the replacement pump, via the communication interface, the infusion program stored in the memory to the replacement pump.

Here and in the following, an insulin pump which has been used or is in use and which is to be replaced is referred to as 'source pump'. An insulin pump which shall be used for continuing the therapy is referred to as 'replacement pump'.

In the following further aspects as well as particular embodiments of the insulin pump as well as its operation are disclosed in more detail. The insulin pump may especially be an insulin pump of an insulin infusion system and may be used in an insulin pump replacement method as described above. Therefore, the disclosed embodiments of the insulin pump according to the invention and its operation also define corresponding embodiments of the insulin pumps in an insulin infusion system and of the method for replacing a first insulin pump by a second insulin pump as described above.

The insulin pump may be configured to perform the steps of:
a) searching for the presence of a source pump, and, when the presence of a source pump is detected, performing the steps of:
b) receiving, via the communication interface, an infusion program from the source pump, and
c) storing the infusion program in the memory.

An insulin pump of this type of embodiment is capable of alternatively serving as both the first insulin pump or the second insulin pump in an insulin infusion system and a replacement method as described above.

For best understanding several aspects of the present invention as well as the interaction between the source pump and the replacement pump, it is generally assumed in the following that the same type of device may be alternatively act as source pump or replacement pump. However, it will be understood by a person skilled in the art that this is not essential as discussed above.

The communication interface via which an infusion program may be transmitted from a source pump to a replacement pump may in particular be a wireless bidirectional communication interface which allows simple data exchange. The communication interface may especially be a radio frequency interface according to a communication standard known in the art, such as the BLUETOOTH standard, or according to a proprietary standard. Alternatively, the wireless bidirectional communication interface may be an infrared interface. In further alternative embodiments, the communication interface is a wired interface.

If a remote controller is present, it is also possible to exchange data with the remote controller via the same communication interface of the insulin pump. Alternatively, the insulin pump may comprise a further separate communication interface for communicating with a remote controller. The insulin pump is advantageously configured to receive its infusion program from a remote controller alternatively to receiving it from a source pump.

In the following sections, major aspects and embodiments of the invention are discussed which are of particular importance for an insulin pump which is acting as source pump.

The control unit is configured to monitor, during operation of the insulin pump according the infusion program, a status of the insulin pump and to activate the search for the presence of a replacement pump when a predetermined status is assumed by the insulin pump.

Searching for a replacement pump only if a predetermined status is assumed is advantageous with respect to energy consumption as well as with respect to the avoidance of handling errors since it prevents an unintended program transfer. The status of the insulin pump is advantageously checked in a test mode which is periodically activated by a timer of the control unit. Alternatively or additionally, the search for a replacement pump may be activated by an interrupt signal which is generated when the predetermined status is assumed. Here, a dedicated test mode is not required.

The status of the insulin pump which is monitored during operation may especially comprise a filling state of the insulin reservoir. A search for another pump to act as replacement pump may be activated if the filing volume of the insulin reservoir falls below a predefined value of, e.g. 10%, of the maximum filling volume. The status may further comprise an error status, such that the occurrence of an error condition, i.e. an occluded infusion cannula or a device error, is periodically monitored and a search for another insulin pump is activated upon the occurrence of an error condition.

The status may further comprise the status of a battery of the insulin pump. This is especially favourable if the battery is integral with the pump and cannot be replaced. While the battery capacity of those devices generally includes a safety factor which ensures that the battery is not exhausted prior to the reservoir being empty, this situation may well occur if the battery is, due improper assembly, partly discharged during assembly of the infusion pump, or due to a defective of the battery itself, or the like.

Therefore, the battery voltage may be monitored by a voltage monitoring unit in an analogue way to the filling volume of the insulin reservoir.

Additionally or alternatively to the voltage, the energy effectively taken from the batter may be monitored by a gauging unit known in the art.

In some embodiments, the source pump transmits the status of the source pump to the replacement pump along with the infusion program. For example, it may transmit an error code of an error which resulted the source pump to activate the search for a replacement pump.

The insulin pump may be designed such that insulin delivery is continued during the search for a replacement pump. If the search is activate, for example, because of the filling state of the insulin reservoir falling below a predefined value as described above, the delivery should be continued until the program is transferred to a replacement pump such that the insulin delivery is not interrupted longer than required.

The control unit may be configured to control the insulin pump to terminate operation after successful transmission of the infusion program to the replacement pump. Terminating operation typically comprises stopping the insulin delivery, followed by switching off the pump or changing to a safe state. It may further comprise an automated rejection of the infusion cannula by an inserter of the insulin pump.

In the following sections, major aspects and embodiments of the invention are discussed which are of particular importance for an insulin pump which is acting as replacement pump.

In embodiments of the insulin pump which allow the insulin pump to act as replacement pump, the control unit may be configured to control the insulin pump, after successfully having received an infusion program from the source pump, to automatically start insulin delivery. Starting the insulin delivery may additionally comprise preparative steps, in particular priming the infusion cannula with insulin and/or inserting the infusion cannula into the patient's subcutaneous tissue by an automatic inserter of the insulin pump. Automatic insertion of the infusion cannula is especially favourable if the insulin pump is a patch pump which is secured to the patient's skin via an adhesive layer.

An insulin pump according to this type of embodiment allows simple replacement or exchange, and, thus, continuation of the therapy, without requiring the presence of a remote controller and without requiring the presence of operation elements, such as buttons or a display, on the pump. Sine all required steps are performed automatically under control of the two pumps, the patient simply secures the newly programmed replacement pump to his or her body after the transfer of the infusion program is complete.

In some embodiments where the infusion cannula of the replacement pump is automatically inserted into the skin, the control unit of the replacement pump is configured to wait, after the replacement pump has successfully received the infusion program, for a defined period of time before actuating the inserter. This defined period of time between the transfer of the program and the insertion of the cannula gives the patient sufficient time to secure the insulin pump to the body. A time period of 10-15 minutes is supposed to be well-suited. In principle, it is also possible to wait longer than 15 minutes for insertion of the cannula. However, the longer the wait until insertion of the cannula, the later the insulin pump is able to start supplying the patient with insulin. A shorter waiting time is possible as well.

As long as the insulin pump which is acting as replacement pump is not in use and has not received an infusion program from a remote controller or a source pump, it is advantageously in a low-energy mode. As described above, in particular disposable pumps are typically provided with a readily built-in battery. Since the size of such a battery is limited by the overall device dimensions and the pump may be stored for several weeks or months prior to its use, minimum energy consumption is highly desirable in the low-energy mode.

In the low energy mode, the insulin pump may be periodically switched into a search mode to detect the presence of a source pump or remote controller as will be discussed below in greater detail. The time interval may, for example, be 1 sec, 1 min or 3 min. In a similar way, an additional device for detecting another insulin pump may be activated periodically in a search mode. Setting the time interval requires a compromise between a short delay for detecting the presence of a source pump on the one hand and low energy consumption on the other hand.

In the following sections, major aspects and embodiments of the invention are discussed which are of particular importance for the communication between a source pump and a second pump and the transfer of the infusion program.

Both an insulin pump acting as source pump as well as an insulin pump acting as replacement pump may operate in a search mode. The term 'search mode' is used for a mode where the pump searches for the presence of an other insulin pump, but a communication line has not yet been established. The search mode may be either active or passive. If the search mode is active, the insulin pump emits signals, such as infrared or radiofrequency signals to be received and responded to by the other insulin pump. If the search mode is passive, the insulin pump waits for the reception of signals emitted by the other insulin pump and responds to such received signals.

As will be discussed below in detail, the insulin pump is advantageously designed such that the search mode is active if the insulin pump serves as source pump and is passive if the insulin pump serves as replacement pump. However, different embodiments may be used as well.

In some embodiments, the communication interface which is used for transmitting the infusion program of the source pump to the replacement pump may also be used for detecting the presence of the other of the source pump and the replacement pump, respectively. Alternatively, the insulin pump has an additional device for detecting another insulin pump. In this way, the detection of another insulin pump can take place independently of the communication interface which is used for the data exchange. While requiring additional hardware, this affords the advantage that the communication interface only has to be activated after the detection of the other insulin pump which is favourable with respect to energy consumption and safety.

For this purpose, it is possible, to use switches or sensors that detect magnetic and/or electric fields. However, purely mechanical switches could also be provided, which may, for example, be activated by a coupling member, such as a notch, of the other insulin pump.

In particular embodiment, the device for detecting another insulin pump comprises a reed relay or a Hall sensor. In particular, a reed relay can be used to detect magnetic fields, that are generated, for example, by a permanent magnet or an electro magnet in the other insulin pump. This represents a particularly energy-efficient solution, since the reed relay itself does not require current, and the energy for changing the switch state is introduced into the insulin pump from outside.

However, it can also be advantageous to use a Hall sensor to detect the other insulin pump. When the Hall sensor has a current passed through it and is brought into a magnetic field running perpendicular to it, it delivers an output voltage. A switch function can thus be obtained in combination with a transistor and/or further semi-conductor components known in the art. In this way, as in the case of the reed relay, the magnetic fields of permanent magnets arranged in the other insulin pump can be detected. The Hall sensor has the advantage that there are no mechanically movable parts that could be damaged, for example, if the insulin pump were accidentally dropped.

For embodiments comprising a magnetic-field sensitive switch or sensor, the device for detecting another insulin pump advantageously further comprises a magnet, such as a permanent or an electro magnet for generating a magnetic field which activates the magnetic field-sensitive switch or sensor in the other insulin pump. By approaching the insulin pumps, the magnetic field generated by the magnet in either of the insulin pumps may activate the magnetic-field sensitive switch or sensor of the other the other insulin pump. The magnet and the magnetic-field sensitive switch or sensor should be arranged shielded from each other or at sufficiently spaced positions of the insulin pump, such that the switch or sensor is not operated by the magnet of the same pump.

The magnet and the magnetic-field sensitive switch or sensor may be designed for an activation distance of some centimetres or for activation only if the insulin pumps touch each other. A well defined orientation of the insulin pumps with respect to each other may further be required for the magnetic-field sensitive switch or sensor to be activated.

In some embodiments, it is sufficient if the magnetic-field sensitive switch or sensor of one of the pumps, for example the replacement pump, is activated by a magnetic field emitted by the other insulin pump. In some of those embodiments, the insulin pump comprises an electro magnet which is powered only in the search mode.

Alternatively, the insulin pump may be designed such that a program transfer may only be performed if a magnetic-field sensitive switch or sensor of both of the two pumps is activated by the other of the two pumps.

In another embodiment, the device for detecting the other insulin pump is designed as an infrared interface as proven and reliable technology. In addition, coded signals can also be transmitted with an infrared interface such that, before the actual data exchange via the bidirectional communication interface, it is possible to verify whether both communicating devices are compatible insulin pumps.

For embodiments comprising a switch which has to be activated before an infusion program can be transferred to it, a remote controller may be configured to activate the switch in the same way as a source pump.

In embodiments comprising a switching element, such as a mechanical switch or a reed relay as device to detect the presence of another insulin pump, the insulin pump may be switched off in the low-energy mode. Only activating the switch results in the controller of the pump to be powered. Once the insulin pump is in operation, the components of the insulin pump may be connected with the battery by a further relay, semiconductor switch, or the like, such that activation of the switch by en external energy is not further required. For this type of embodiment, the insulin pump does not consume any energy in the low-energy mode since it does not need to periodically activate a communication interface or a device for detecting another insulin pump since the energy for switching the switch and, thus, changing the operation mode of the insulin pump is introduced into the insulin pump from outside, that is, from the other insulin pump.

If the insulin pump comprises an electro magnet for activating a magnetic-field sensitive switch of another insulin pump, it is advantageously designed such the electro magnet of the source pump is powered in the search mode, while the electro magnet of the replacement pump is not powered in the low-energy mode and search mode. This kind of embodiment is advantageous since it requires the replacement pump not to emit energy in the low energy mode and thus involves minimum energy consumption of the unused replacement pump prior to its application. Only after the magnetic-field sensitive switch or sensor of the receiving pump has been activated by the source pump, it may optionally power its electro magnet to activate the magnetic-field sensor or switch of the source pump for acknowledging purposes.

As stated above, the insulin reservoir of the pump is typically in an initial empty state and is filled by the patient prior to use. In some advantageous embodiments, the insulin pump may be designed such that filling of the insulin reservoir is automatically detected. Upon detecting of the insulin reservoir being filled, the insulin pump advantageously switches to the search mode. If the pump is of the syringe-driver type, a plug of the insulin reservoir is advantageously in an initial most proximal position corresponding to an empty drug reservoir and is displaced into a distal position if fluid is forced into the insulin reservoir, e.g., via a syringe. The pump may be designed such that this displacement of the plug operates an electrical contact which triggers the pump to switch to the search mode. Before the contact is operated, the insulin pump may be switched off.

In some embodiments, the insulin pump comprises indicators such as light emitting diodes, a buzzer or loudspeaker, a pager vibrator, or the like. Those indicators are advantageously used to indicate one or multiple of a number of situations. In particular, the indicators may indicate least one of the search for the replacement pump, the successful detection of the replacement pump, the successful transmission of the program to the replacement pump, or the unsuccessful transmission of the program to the replacement pump.

In embodiments of the insulin pump which may act as replacement pump, an indication unit may be configured to indicate at least one of the search for the source pump being active, the establishment of communication with the source pump, the successful reception of the infusion program from the replacement pump, the unsuccessful reception of the infusion program from the replacement pump, or the start of insulin delivery.

Further advantageous embodiments and detailed aspects of the invention may be derived from exemplary embodiments as described below in more detail.

### Brief description of the drawings

In the drawings used to explain the illustrative embodiment:
- Fig.1: schematically shows the replacement procedure of a first insulin pump by a second insulin pump according to the invention.
- Fig. 2: shows a plan view of the rear face of an insulin pump according to the invention;
- Fig. 3: shows a side view of the insulin pump from Fig. 1;
- Fig. 4: shows a schematic view of the inner area and of the functional elements of the insulin pump in a cross section along the line A-B in Fig. 2;
- Fig. 5: shows a schematic sequence of deactivation of a source pump which is in delivery mode, with the insulin reservoir almost used up, and which is exchanged for a new replacement pump;
- Fig. 6: shows a schematic sequence of the steps involved in the activation of the new replacement pump.

Identical parts in the figures are in principle provided with identical reference numbers.

### Ways of implementing the invention

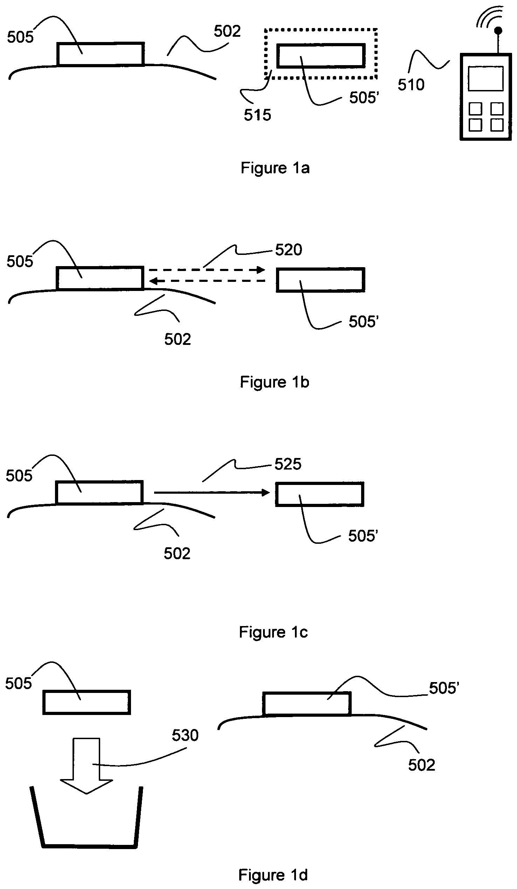

Figure 1a shows a first insulin pump 505 in accordance with the present invention which is attached to the skin 502 of a patient and is in the delivery mode. The first insulin pump 505 may have received its infusion program from a remote controller 510 or from a previously used insulin pump in the same way as described below. The additional infusion of insulin boli as well as further programming and control capabilities for the first insulin pump 505 are available via the remote controller 510. Figure 1b further shows a second insulin pump 505' which is of the same type as the first insulin pump 500. The second insulin pump 505' is provided in a sterile box 515 and is in a low-energy mode or sleeping mode.

Figure 1b shows a later situation where a status of the first insulin pump 505 is such that it may be replaced due to the filling state of its insulin reservoir, the status of the battery or the occurrence of an error condition. The first insulin pump 505 gives an indication to the patient via an acoustical and/or optical indicator (not visible in Fig. 1) and activates the search for a second insulin pump as described below in more detail. Upon the indication, the patient removes the second insulin pump 505' from its sterile box 515 and approaches it to the first insulin pump 505. Prior to performing this step, he may fill the insulin reservoir of the replacement pump 505' if it was initially empty. In the following, the first insulin pump 505 acts as source pump while the second insulin pump 505' acts as replacement pump. The presence of the replacement pump 505' is detected by the source pump 500 and communication is established as indicated by the arrows 520.

After establishing the communication, the source pump 505 transfers the infusion program to the replacement pump 505' as indicated in Figure 1c by arrow 525. After successful transmission of the infusion program, the source pump 505 terminates its operation.

The final steps of the replacement procedure are shown in Figure 1d. The patient removes the source pump 505 from its skin 502 ad discards it, as indicated by arrow 530. The patient attaches the replacement pump 505' to its skin 502. After a delay of some minutes, the replacement pump 505' may carry out preparative steps, in particular priming its infusion cannula with insulin and inserting the infusion cannula into the patient's subcutaneous tissue and subsequently starts insulin infusion. If the insulin pumps do not comprise an infusion cannula which is automated inserted into the patient's tissue, this step is carried out manually by the patient.

Further aspects, variations and modifications of this procedure will become more readily visible in the following in the framework of the exemplary embodiments of insulin pumps according to the invention.

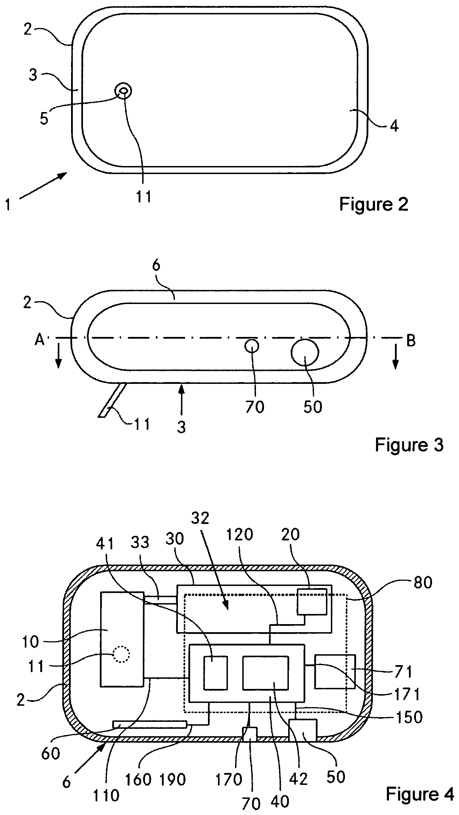

An insulin pump 1 according to the invention is shown in different views in Figures 2-4. It comprises a housing 2 in the form of a hollow parallelepipedal body with rounded corners and edges.

Figure 2 is a view of the underside 3 of the insulin pump 1. The underside 3 of the housing 2 is substantially rectangular and has rounded corners. Except for a narrow edge area, the underside 3 is also covered with an adhesive coating 4, which is provided for securing the insulin pump 1 to the body of a patient, e.g. in the abdominal area. Moreover, a circular opening 5 is formed in the underside 3 of the housing 2, more or less centrally on the left-hand side. This opening 5 serves as a passage for the cannula 11 arranged in the automatic inserter 10 in the interior of the housing 2. The automatic inserter 10 is designed such that it can deploy the cannula 11 from a completely retracted position in the interior of the housing 2 and through the opening 5, in an oblique direction with respect to the underside 3, several millimetres from the opening 5.

In Figure 3, which represents a side view of a first lengthwise side 6 of the insulin pump 1, the cannula 11 is shown in the fully deployed position. The cannula 11 is typically made from a soft and biocompatible material such as Teflon and protrudes down to the left in an oblique direction with respect to the underside 3 of the insulin pump 1. Moreover, in a lower area at the right of the lengthwise side 6 of the housing 2, an infrared interface 50 is arranged in a recess of the housing 2 and is used to detect another insulin pump. To the left alongside it, an optional light-emitting diode 70 is located as a status indicator in another recess of the housing 2.

In Figure 4, the inner structure of the insulin pump 1 is shown schematically in a cross section through the housing 2 of the insulin pump 1 along the line A-B from Figure 3. The automatic inserter 10 is arranged on the left-hand side, and the inserter 10 communicates with an insulin reservoir 30 via a tube 33. The insulin reservoir 30 itself is arranged in an upper edge area along the upper and second lengthwise side of the housing 2. Inside the automatic inserter 10, there is a tubing system (not shown) connecting the cannula 11 to the tube 33. The insulin 32 present in the insulin reservoir 30 can be conveyed to the cannula 11 through the tube 33 and the automatic inserter 10 by means of a delivery device 20 coupled to the insulin reservoir. The delivery device 20 comprises, for example, a plug (not shown in Figure 4) which is moved or forced into the insulin reservoir 30 by a spindle mechanism, thus displacing a plug of the insulin reservoir. More details on the general design of such an insulin pump are found in EP 0 991 440 B1, for example. The insulin 32 located in the insulin reservoir 30 is displaced by the plug and, as has been described above, is conveyed through the tube 33 into the cannula 11. In order to determine the filling state of the insulin reservoir 30, it is possible to count the control impulses sent to a stepping motor of the spindle drive and/or to provide a motor with an encoder.

Moreover, a sound generator 71, such as a loudspeaker or buzzer, is integrated in the area before the right-hand narrow side of the housing 2 as additional status indicator and for alarming purposes. The infrared interface 50, already described with respect to Figure3, is arranged on the lower and first lengthwise side 6, in a suitable recess in the housing 2 to the right of the centre. To its left, the light-emitting diode 70, arranged on the lower lengthwise side, is also arranged in a corresponding further recess in the housing 2. To the left of the centre of the lower and first lengthwise side 6, a radiofrequency interface 60 is also arranged in the interior of the housing 2.

A control unit 40 with a microprocessor or microcontroller 41 and a memory unit 42 is arranged in a central area in the interior of the insulin pump 1. The control unit 40 is connected to the inserter via a first control line 110, such that the insertion and optionally also the retraction of the cannula 11 can be controlled by the control unit 40. A second control line 120 between control unit 40 and delivery device 20 permits the transfer of control impulses from the control unit 40 to the delivery device 20. The sound generator 71 is connected by a third control line 171 to the control unit 40 and can thus be activated by the latter. The light-emitting diode 70 is likewise connected by a fourth control line 170 to the control unit 40 and can be switched on or off by the latter. The fifth control line 150 between the infrared interface 50 and the control unit 40 permits the activation and deactivation of the infrared interface 50 and also the data transfer between infrared interface 50 and control unit 40. The radiofrequency interface 60 is connected to the control unit 40 by the sixth control line 160. Between control unit 40 and radiofrequency interface 60, data can be transferred and the radiofrequency interface 60 can be activated or deactivated.

Moreover, above the sectional plane shown in Figure 4, there is a battery 80 for the insulin pump 1 (indicated by the broken line). The cabling of the battery 80 is not shown but is configured in a manner known per se.

The memory unit 42 is provided both for storage of the infusion program and also for holding a protocol file or log file of the insulin pump. Selected operating parameters of the insulin pump are recorded in the protocol file.

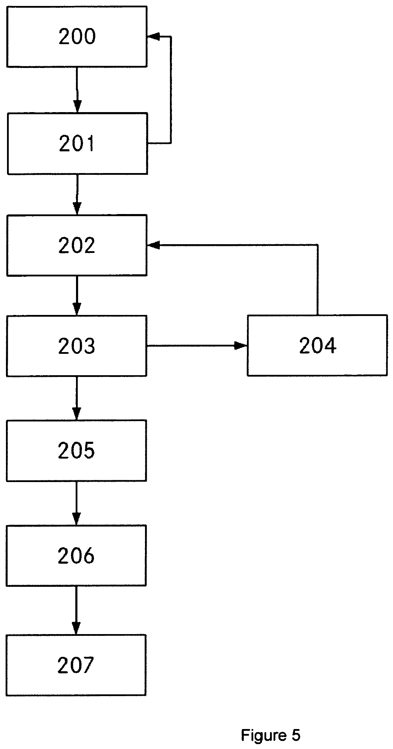

Figure 5 shows the way in which the insulin pump 1 functions as source pump. The insulin pump 1 is first in the delivery mode 200. The insulin pump 1 delivers insulin 32 via the delivery device 20 and supplies the patient with the required doses of insulin 32 in accordance with a basal delivery schedule which is stored as infusion program in the memory unit 42. On the basis of the number of control impulses already sent to the delivery device 20, the control unit 40 additionally determines the filling state of the insulin 32 in the insulin reservoir 30. The light-emitting diode 70 can be additionally used to signal the delivery mode.

In the subsequent test mode 201, the determined filling state of the insulin reservoir is compared to a minimum value stored in the memory unit 42. If the filling state is greater than or equal to the minimum value, the operation is changed back to the delivery mode 200 and the filling state is determined again after a period of time and/or after a following insulin delivery. However, if the control unit 40 determines in the test mode 201 that the filling state is less than the minimum value stored in the memory unit 42, the first interface activation 202 takes place in which the infrared interface 50 is switched on. In the same way, the occurrence of an error condition, such as a device error or an occluded cannula, is monitored in the test mode 201. Optionally, the state of the battery 80 is also determined and compared to a minimum value in an analogue way to the insulin reservoir.

The control unit 40 then changes to the search mode 203, in which the insulin pump 1 searches for an infrared signal from a second and unused replacement pump. If no such signal is detected, the control unit 40 changes to the interface deactivation mode 204. In the interface deactivation mode 204, the infrared interface 50 is first switched off and then a period of 2 minutes, for example, is allowed to elapse before the control unit 40 performs the first interface activation 202 again.

As soon as an infrared signal of a replacement pump is detected during the search mode 203, the insulin pump 1 tests whether the infrared signal has a known and valid code. If no code is recognized, the insulin pump 1 changes back to the interface deactivation mode 204. However, if the code of the infrared signal is recognized by the insulin pump 1, the control unit 40 changes to the connection mode 205. An acknowledgement signal is sent to the replacement pump via the infrared interface 50, and the radiofrequency interface 60 is then switched on. A connection to the replacement pump detected by the infrared interface 50 is then set up via the radiofrequency interface 60. Among other things, the data transfer rates may be established on the basis of the signal quality.

In the subsequent transmit mode 206, the infusion program stored in the memory 42 of the insulin pump 1 is transferred to the replacement pump via the radiofrequency interface 60.

After the data transfer has taken place, the insulin pump 1 changes to the switch-off mode 207 in which operation of the insulin pump 1 is terminated. In the switch-off mode 207, a standby time, for example of 2 minutes, is allowed to elapse. The control unit 40 then deactivates the delivery device 20 and thus stops the delivery of insulin 32. Thereafter, the control unit 40 optionally causes the inserter 10 to retract the cannula 11. The control unit 40 emits an acoustic signal via the sound generator 71 to indicate termination of the operation and switches off all the other components of the insulin pump 1. The insulin pump 1 can thus be removed by the patient from his or her body and, for example, replaced by the replacement pump.

The insulin pump 1 supplies the patient with insulin throughout all the method steps or modes 200-206 shown in Fig.2, except in the switch-off mode 207. In case of an error, the insulin delivery may be terminated and the insulin pump may emit an alert signal via the sound generator 71 and/or the light-emitting diode 70 throughout the modes 200-206.

Alternatively, insulin supply may be stopped between establishing the connection in step 205 and transmitting the program in step 206.

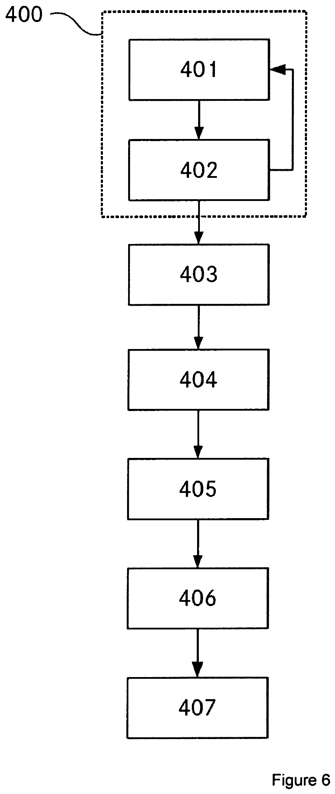

Figure 6 shows the mode of operation of the insulin pump 1 as replacement pump in the unused state and upon first activation. After production, the insulin pump 1 is in the low-energy mode 400 and does not yet store a patient-specific program. In a first step 401, a coded infrared signal is emitted via the infrared interface 50 in the low-energy mode 400. In the following second step 402 of the low-energy mode 400, the infrared interface 50 searches for an acknowledgement signal from another insulin pump (which acts as source pump and accordingly has a patient-specific program). If no acknowledgement signal is detected, the first step 401 of the low-energy mode 400 is performed again. The steps 401 and 402, form in combination, a search mode of the insulin pump 1. The steps may optionally be performed only after filling of the insulin reservoir 30 of the insulin pump 1 by the patient has been detected. The low-energy mode is equivalent with the search mode for this embodiment.

As soon as an acknowledgement signal is detected in the second step 402 of the low-energy mode 400, the control unit 40 activates the radiofrequency interface 60 in the switch-on step 403. A connection is then set up with the other insulin pump detected by the infrared interface 50 and, among other things, the data transfer rate is established on the basis of the signal quality.

In the subsequent receive mode 404, the program of the other insulin pump is transferred to the insulin pump 1 via the radiofrequency interface 60 and stored in the memory unit 42. The program includes in particular the time and date as well as the basal delivery schedule.

Thereafter, the control unit 40 changes to standby mode 405, during which the insulin pump 1 shows no activity for a period of 15 minutes, for example. The standby mode 405 gives a patient sufficient time to apply the insulin pump 1 to the desired location on the body. After the 15 minutes have passed, the insulin pump changes automatically to the use mode 406, in which the control unit 40 causes the inserter 10 to deploy the cannula 11 and thus insert it into the patient's body. Before changing to the standby mode 405, the cannula 11 may optionally be automatically primed in order to displace the air in the cannula 11 by insulin.

The control unit 40 then changes to start-up mode 407. The delivery device 20 is thereby activated by the control unit 40 and delivers insulin 32 from the insulin reservoir 30 into the cannula 11 according to the program stored in the memory unit 42. The control unit 40 also emits an acoustic signal via the sound generator 71 and optionally indicates beginning of the delivery via the light-emitting diode 70. The control unit then changes to the delivery mode 200 described in Fig. 5.

The described embodiment is to be understood as an illustrative example that can be broadened or modified as desired within the context of the invention.

With respect to Figure 5, the insulin pump, may, in the search mode 203, emit an infrared signal to be detected by a replacement pump rather than searching for an infrared signal emitted by a replacement pump, followed by searching for the reception of an acknowledgement signal to be sent by a replacement pump.

In an analogue way, with respect to Figure 6, the insulin pump 1 may, in the mode 401, detect if an infrared signal is received from a source pump rather than emitting a signal. In this case, the step 402 may be omitted. An acknowledgement signal is, in this case, advantageously emitted by the insulin pump 1 in the switch-on-step 403.

Other devices for providing signals and information to the user can be used instead of or in addition to the light-emitting diode 70 or the sound generator 71. For example, the sound generator 71 can be replaced by a tactile element, such as a mechanical vibrator. It is also conceivable to provide a screen for displaying symbols or texts which, for example, provide information on the operating state of the insulin pump.

It is also possible to do without the infrared interface 50 for detection of a replacement pump and to provide a reed relay or a Hall sensor, for example. In this embodiment, a permanent magnet is preferably also accommodated in the interior of the housing 2, such that the insulin pump 1 can also be detected by a replacement pump. Advantageously, the reed relay or Hall sensor is activated only when a filling state of an insulin reservoir of the source pump falls below a predefined minimum value, in particular 10%, and/or on the occurrence of an error condition or another predetermined status of the source pump.. The risk of inadvertent actuation through magnetic interference sources is thereby reduced.

Another communication interface can be provided instead of the radiofrequency interface 60. For example, an infrared interface can also be used for transfer of the program. In principle, however, it is also possible to provide just one communication interface, for example the radiofrequency interface 60, which serves both for detection and also for transfer of the program.

The shape of the housing 2 can of course also have a more ergonomic form, such that the insulin pump 1 is easier to handle and protrudes as little as possible on the patient's body.

It is also possible to determine the filling state of the insulin reservoir 30 by using a filling state sensor arranged on the insulin reservoir 30. By way of a control line connecting the filling state sensor to the control unit 40, the filling state determined in the insulin reservoir 30 by the filling state sensor can be transmitted to the control unit 40.

It is also possible that the acknowledgement signal received in the second step 402 of the low-energy mode 400 originates from a remote controller and, accordingly, the program is transferred from the operating appliance to the insulin pump in the receive mode. This is the normal case where an operational remote controller is present.

### List of reference numbers

- 1: insulin pump
- 2: housing
- 3: underside
- 4: adhesive coating
- 5: opening in underside
- 6: first lengthwise side
- 10: inserter
- 11: cannula
- 20: delivery device
- 30: insulin reservoir
- 32: insulin
- 33: tube
- 40: control unit
- 41: microprocessor
- 42: memory
- 50: infrared interface
- 60: radiofrequency interface
- 70: light-emitting diode
- 71: sound generator with loudspeaker
- 80: battery
- 110: first control line (40 - 10)
- 120: second control line (40 - 20)
- 150: fifth control line (40 - 50)
- 160: sixth control line (40 - 60)
- 170: fourth control line (40 - 70)
- 171: third control line (40 - 71)
- 200: operating mode
- 201: test mode
- 202: first interface activation
- 203: search mode
- 204: interface deactivation mode
- 205: connection mode
- 206: search mode
- 207: switch-off mode
- 400: low-energy mode
- 401: first step of 400
- 402: second step of 400
- 403: switch-on step
- 404: receive mode
- 405: standby mode
- 406: use mode
- 407: start-up mode
- 502: skin
- 505: first insulin pump (source pump)
- 505': second insulin pump (replacement pump)
- 510: remote controller
- 515: sterile box
- 520: communication establishment
- 525: infusion program transmission
- 530: discarding

## Claims

1. Insulin delivery system, the insulin delivery system comprising a first insulin pump (1, 505) and a second insulin pump (1, 505'), each of the first insulin pump (1, 505) and the second insulin pump (1, 505') comprising:
a) a memory (42) which is configured to store an infusion program,
b) a control unit (40) which is configured to control operation of the insulin pump (1, 505, 505'),
c) a communication interface (60) for data exchange with the other of the first insulin pump (1, 505) and the second insulin pump (1, 505'),
the control unit (40) of the first insulin pump (1, 505) being configured to autonomously perform the steps of
d) monitoring, during operation of the first insulin pump (1, 505) according to the infusion program, a status of the first insulin pump (1, 505), wherein the status comprises at least one of a filling state of an insulin reservoir (30) of the insulin pump (1, 505), the status of a battery (80) of the insulin pump (1, 505), and an error status of the insulin pump (1, 505),
e) activating a search for the presence of the second insulin pump (1, 505') when a predetermined status is assumed by the first insulin pump (1, 505), and, upon detection of the presence of the second insulin pump (1, 505'),
f) transmitting, via the communication interface (60) of the first insulin pump (1, 505), the infusion program stored in the memory (42) of the first insulin pump (1, 505) to the second insulin pump (1, 505'),
the control unit (40) of the second insulin pump (1, 505') being configured to perform the steps of
g) searching for the presence of the first insulin pump (1, 505), and, upon detection of the presence of the first insulin pump (1, 505),
h) receiving, via the communication interface (60) of the second insulin pump (1, 505'), an infusion program from the first insulin pump (1, 505), and
i) storing the infusion program in the memory (42) of the second insulin pump (1, 505').

2. The insulin delivery system as claimed in claim 1, **characterized in that** the first insulin pump (1, 505) and the second insulin pump (1, 505') are identical insulin pumps.

3. Insulin pump (1,505), comprising:
a) a communication interface (60) for data exchange with a replacement pump (1, 505'),
b) a memory (42) which is configured to store an infusion program,
c) a control unit (40) which is configured to control operation of the insulin pump (1, 505) and which is further configured to transmit, upon detecting the presence of a replacement pump, via the communication interface (60), the infusion program stored in the memory (42) to the replacement pump (1, 505'),
wherein the control unit (40) is configured to autonomously perform the steps of
d) monitoring, during operation of the insulin pump (1, 505) according to the infusion program, a status of the insulin pump (1, 505), wherein the status comprises at least one of a filling state of an insulin reservoir (30) of the insulin pump (1, 505), the status of a battery (80) of the insulin pump (1, 505), and an error status of the insulin pump (1, 505),
e) activating a search for the presence of a replacement pump (1, 505') when a predetermined status is assumed by the insulin pump (1, 505), and
f) transmitting, upon detecting the presence of the replacement pump, via the communication interface (60), the infusion program stored in the memory (42) to the replacement pump (1, 505').

4. Insulin pump (1, 505) as claimed in claim 3, **characterized in that** the control unit (40) is configured to control the insulin pump (1, 505) to continue insulin delivery while a search for the presence of the replacement pump (1, 505') is active.

5. Insulin pump as claimed in either of claim 3 or claim 4, **characterized in that** the control unit (40) is configured to control the insulin pump (1, 505) to terminate operation after successful transmission of the infusion program to the replacement pump (1, 505').

6. The insulin pump (1, 505) as claimed in either of claim 3 to claim 5, **characterized in that** the insulin pump (1, 505) has, in addition to the wireless communication interface (60), a device (50) for detecting the presence of the replacement pump (1, 505').

7. The insulin pump as claimed in either claim 3 to claim 6, **characterized in that** the insulin pump (1, 505) is configured to receive the infusion program from a remote controller (510).

8. The insulin pump as claimed in either of claim 3 to claim 7, **characterized in that** the control unit (40) is configured to indicate, via an indicator (70, 71) of the insulin pump (1, 505), at least one of the search for the replacement pump (1, 505'), the successful detection of the replacement pump (1, 505'), the successful transmission of the program to the replacement pump (1, 505'), or the unsuccessful transmission of the program to the replacement pump (1, 505').

9. The insulin pump as claimed in either claim 3 to claim 8, **characterized in that** the insulin pump (1, 505') is configured to perform the steps of:
a) searching for the presence of a source pump (1, 505), and, when the presence of a source pump is detected, performing the steps of:
b) receiving, via the communication interface (60), an infusion program from the source pump (1, 505), and
c) storing the infusion program in the memory (42).

10. The insulin pump (1, 505') as claimed in claim 9, **characterized in that** the control unit (40) is configured to control the insulin pump (1), after successfully having received an infusion program from the source pump (1, 505), to autonomously start insulin delivery.

11. The insulin pump as claimed in either of claim 9 or claim 10, **characterized in that** the control unit (40) is configured to detect an insulin reservoir (30) of the insulin pump (1, 505') being filled, and, upon the insulin reservoir (30) being filled, to activate the search for a source pump (1, 505).

## Patentansprüche

1. Insulinverabreichungssystem, wobei das Insulinverabreichungssystem eine erste Insulinpumpe (1, 505) und eine zweite Insulinpumpe (1, 505') umfasst, wobei jede der ersten Insulinpumpe (1, 505) und der zweiten Insulinpumpe (1, 505') umfasst:
a) einen Speicher (42), der konfiguriert ist, um ein Infusionsprogramm zu speichern,
b) eine Steuereinheit (40), die konfiguriert ist, um den Betrieb der Insulinpumpe (1, 505, 505') zu steuern,
c) eine Kommunikationsschnittstelle (60) zum Datenaustausch mit der anderen von der ersten Insulinpumpe (1, 505) und der zweiten Insulinpumpe (1, 505'),
wobei die Steuereinheit (40) der ersten Insulinpumpe (1, 505) konfiguriert ist, um die folgenden Schritte autonom durchzuführen
d) Überwachen eines Zustands der ersten Insulinpumpe (1, 505) während des Betriebs der ersten Insulinpumpe (1, 505) gemäß dem Infusionsprogramm, wobei der Zustand mindestens einen von einem Füllzustand eines Insulinreservoirs (30) der Insulinpumpe (1, 505), dem Zustand einer Batterie (80) der Insulinpumpe (1, 505) und einem Fehlerzustand der Insulinpumpe (1, 505) umfasst,
e) Aktivieren einer Suche nach dem Vorhandensein der zweiten Insulinpumpe (1, 505'), wenn ein vorgegebener Zustand durch die erste Insulinpumpe (1, 505) angenommen wird, und, nach dem Erfassen des Vorhandenseins der zweiten Insulinpumpe (1, 505'),
f) Übertragen des in dem Speicher (42) der ersten Insulinpumpe (1, 505) gespeicherten Infusionsprogramms über die Kommunikationsschnittstelle (60) der ersten Insulinpumpe (1, 505) an die zweite Insulinpumpe (1, 505'),
wobei die Steuereinheit (40) der zweiten Insulinpumpe (1, 505') konfiguriert ist, um die folgenden Schritte durchzuführen
g) Suchen nach dem Vorhandensein der ersten Insulinpumpe (1, 505) und, nach dem Erfassen des Vorhandenseins der ersten Insulinpumpe (1, 505),
h) Empfangen eines Infusionsprogramms von der ersten Insulinpumpe (1, 505') über die Kommunikationsschnittstelle (60) der zweiten Insulinpumpe (1, 505'), und
i) Speichern des Infusionsprogramms in dem Speicher (42) der zweiten Insulinpumpe (1, 505').

2. Insulinverabreichungssystem nach Anspruch 1, **dadurch gekennzeichnet, dass** die erste Insulinpumpe (1, 505) und die zweite Insulinpumpe (1, 505') identische Insulinpumpen sind.

3. Insulinpumpe (1, 505), umfassend:
a) eine Kommunikationsschnittstelle (60) zum Datenaustausch mit einer Ersatzpumpe (1, 505'),
b) einen Speicher (42), der konfiguriert ist, um ein Infusionsprogramm zu speichern,
c) eine Steuereinheit (40), die zum Steuern des Betriebs der Insulinpumpe (1, 505) konfiguriert ist und die ferner konfiguriert ist, um bei Erfassen des Vorhandenseins einer Ersatzpumpe über die Kommunikationsschnittstelle (60) das im Speicher (42) gespeicherte Infusionsprogramm an die Ersatzpumpe (1, 505') zu übertragen,
wobei die Steuereinheit (40) konfiguriert ist, um die folgenden Schritte autonom durchzuführen
d) Überwachen eines Zustands der Insulinpumpe (1, 505) während des Betriebs der Insulinpumpe (1, 505) gemäß dem Infusionsprogramm, wobei der Zustand mindestens eines von einem Füllzustand eines Insulinreservoirs (30) der Insulinpumpe (1, 505), dem Zustand einer Batterie (80) der Insulinpumpe (1, 505) und einem Fehlerzustand der Insulinpumpe (1, 505) umfasst,
e) Aktivieren einer Suche nach dem Vorhandensein einer Ersatzpumpe (1, 505'), wenn ein vorgegebener Zustand durch die Insulinpumpe (1, 505) angenommen wird, und
f) Übertragen des in dem Speicher (42) gespeicherten Infusionsprogramms bei Erfassung des Vorhandenseins der Ersatzpumpe über die Kommunikationsschnittstelle (60) an die Ersatzpumpe (1, 505').

4. Insulinpumpe (1, 505) nach Anspruch 3, **dadurch gekennzeichnet, dass** die Steuereinheit (40) zum Steuern der Insulinpumpe (1, 505) konfiguriert ist, um die Insulinverabreichung fortzusetzen, während eine Suche nach dem Vorhandensein der Ersatzpumpe (1, 505') aktiv ist.

5. Insulinpumpe nach Anspruch 3 oder Anspruch 4, **dadurch gekennzeichnet, dass** die Steuereinheit (40) zum Steuern der Insulinpumpe (1, 505) konfiguriert ist, um den Betrieb nach erfolgreicher Übertragung des Infusionsprogramms an die Ersatzpumpe (1, 505') zu beenden.

6. Insulinpumpe (1, 505) nach einem der Ansprüche 3 bis 5, **dadurch gekennzeichnet, dass** die Insulinpumpe (1, 505) zusätzlich zu der drahtlosen Kommunikationsschnittstelle (60) eine Vorrichtung (50) zum Erfassen des Vorhandenseins der Ersatzpumpe (1, 505') aufweist.

7. Insulinpumpe nach einem der Ansprüche 3 bis 6, **dadurch gekennzeichnet, dass** die Insulinpumpe (1, 505) konfiguriert ist, um das Infusionsprogramm von einer Fernsteuereinrichtung (510) zu empfangen.

8. Insulinpumpe nach einem der Ansprüche 3 bis 7, **dadurch gekennzeichnet, dass** die Steuereinheit (40) konfiguriert ist, um über eine Anzeigevorrichtung (70, 71) der Insulinpumpe (1, 505) mindestens eines von der Suche nach der Ersatzpumpe (1, 505'), der erfolgreichen Erfassung der Ersatzpumpe (1, 505'), der erfolgreichen Übertragung des Programms an die Ersatzpumpe (1, 505') oder der erfolglosen Übertragung des Programms an die Ersatzpumpe (1, 505') anzuzeigen.

9. Insulinpumpe nach einem der Ansprüche 3 bis 8, **dadurch gekennzeichnet, dass** die Insulinpumpe (1, 505') konfiguriert ist, um die folgenden Schritte durchzuführen:
a) Suchen nach dem Vorhandensein einer Ursprungspumpe (1, 505), und, wenn das Vorhandensein einer Ursprungspumpe erkannt wird, Durchführen der folgenden Schritte:
b) Empfangen eines Infusionsprogramms von der Ursprungspumpe (1, 505) über die Kommunikationsschnittstelle (60) und
c) Speichern des Infusionsprogramms in dem Speicher (42) .

10. Insulinpumpe (1, 505') nach Anspruch 9, **dadurch gekennzeichnet, dass** die Steuereinheit (40) konfiguriert ist, um die Insulinpumpe (1) zu steuern, nachdem sie erfolgreich ein Infusionsprogramm von der Ursprungspumpe (1, 505) empfangen hat, um die Insulinzufuhr autonom zu starten.

11. Insulinpumpe nach Anspruch 9 oder Anspruch 10, **dadurch gekennzeichnet, dass** die Steuereinheit (40) konfiguriert ist, um ein Insulinreservoir (30) der Insulinpumpe (1, 505'), das gefüllt wird, zu erfassen und beim Füllen des Insulinreservoirs (30) die Suche nach einer Ursprungspumpe (1, 505) zu aktivieren.

## Revendications

1. Système de délivrance d'insuline, ce système de délivrance d'insuline comprenant une première pompe à insuline (1, 505) et une seconde pompe à insuline (1, 505'), chacune de la première pompe à insuline (1, 505) et de la seconde pompe à insuline (1, 505') comprenant
a) une mémoire (42) qui est configurée pour sauvegarder un programme de perfusion,
b) une unité de commande (40) qui est configurée pour commander le fonctionnement de la pompe à insuline (1, 505, 505'),
c) une interface de communication (60) pour l'échange de données avec l'autre parmi la première pompe à insuline (1, 505) et la seconde pompe à insuline (1, 505'),
l'unité de commande (40) de la première pompe à insuline (1, 505) étant configurée pour réaliser de manière autonome les étapes de
d) contrôle, pendant le fonctionnement de la première pompe à insuline (1, 505) suivant le programme de perfusion, d'un état de la première pompe à insuline (1, 505), cet état comprenant au moins soit un état de remplissage d'un réservoir à insuline (30) de la pompe à insuline (1, 505), soit un état d'une batterie (80) de la pompe à insuline (1, 505), soit un état d'erreur de la pompe à insuline (1, 505),
e) activation d'une recherche de la présence de la seconde pompe à insuline (1, 505') lorsqu'un état prédéterminé est pris par la première pompe à insuline (1, 505) et, en cas de détection de la présence de la seconde pompe à insuline (1, 505'),
f) transmission, via l'interface de communication (60) de la première pompe à insuline (1, 505), du programme sauvegardé dans la mémoire (42) de la première pompe à insuline (1, 505) à la seconde pompe à insuline (1, 505'),
l'unité de commande (40) de la seconde pompe à insuline (1, 505') étant configurée pour réaliser les étapes de
g) recherche de la présence de la première pompe à insuline (1, 505) et, en cas de détection de la présence de la première pompe à insuline (1, 505),
h) réception, via l'interface de communication (60) de la seconde pompe à insuline (1, 505'), d'un programme de perfusion en provenance de la première pompe à insuline (1, 505), et
i) sauvegarde du programme de perfusion dans la mémoire (42) de la seconde pompe à insuline (1, 505') .

2. Système de délivrance d'insuline selon la revendication 1, **caractérisé en ce que** la première pompe à insuline (1, 505) et la seconde pompe à insuline (1, 505') sont des pompes à insuline identiques.

3. Pompe à insuline (1, 505), comprenant :
a) une interface de communication (60) pour l'échange de données avec une pompe de remplacement (1, 505'),
b) une mémoire (42) qui est configurée pour sauvegarder un programme de perfusion,
c) une unité de commande (40) qui est configurée pour commander le fonctionnement de la pompe à insuline (1, 505) et qui est en outre configurée pour transmettre, en cas de détection de la présence d'une pompe de remplacement, via l'interface de communication (60), le programme de perfusion sauvegardé dans la mémoire (42) à la pompe de remplacement (1, 505'),
l'unité de commande (40) étant conçue pour réaliser de manière autonome les étapes de
d) contrôle, pendant le fonctionnement de la pompe à insuline (1, 505) suivant le programme de perfusion, d'un état de la pompe à insuline (1,505), cet état comprenant au moins soit un état de remplissage d'un réservoir à insuline (30) de la pompe à insuline (1, 505), soit un état d'une batterie (80) de la pompe à insuline (1, 505), soit un état d'erreur de la pompe à insuline (1, 505),
e) activation d'une recherche de la présence d'une pompe de remplacement (1, 505') lorsqu'un état prédéterminé est pris par la pompe à insuline (1, 505) et,
f) transmission, en cas de détection de la présence de la pompe de remplacement, via l'interface de communication (60), du programme de perfusion sauvegardé dans la mémoire (42) à la pompe de remplacement (1, 505').

4. Pompe à insuline (1, 505) selon la revendication 3, **caractérisée en ce que** l'unité de commande (40) est configurée pour commander la pompe à insuline (1, 505) pour continuer la délivrance d'insuline pendant qu'une recherche de la présence de la pompe de remplacement (1, 505') est active.

5. Pompe à insuline (1, 505) selon soit la revendication 3, soit la revendication 4, **caractérisée en ce que** l'unité de commande (40) est configurée pour commander la pompe à insuline (1, 505) pour mettre fin au fonctionnement après la réussite de la transmission du programme de perfusion à la pompe de remplacement (1, 505') .

6. Pompe à insuline (1, 505) selon soit la revendication 3, soit la revendication 5, **caractérisée en ce que** la pompe à insuline (1, 505) comporte, en plus de l'interface de communication sans fil (60), un dispositif (50) de détection de la présence de la pompe de remplacement (1, 505').

7. Pompe à insuline (1, 505) selon soit la revendication 3, soit la revendication 6, **caractérisée en ce que** la pompe à insuline (1, 505) est configurée pour recevoir le programme de perfusion en provenance d'une commande distante (510).

8. Pompe à insuline selon soit la revendication 3, soit la revendication 7, **caractérisée en ce que** l'unité de commande (40) est configurée pour indiquer, via un indicateur (70, 71) de la pompe à insuline (1, 505), au moins soit la recherche de la pompe de remplacement (1, 505'), soit la détection effective de la pompe de remplacement (1, 505'), soit la réussite de la transmission du programme à la pompe de remplacement (1, 505'), soit l'échec de la transmission du programme à la pompe de remplacement (1, 505').

9. Pompe à insuline selon soit la revendication 3, soit la revendication 8, **caractérisée en ce que** la pompe à insuline (1, 505') est configurée pour réaliser les étapes de :
a) recherche de la présence d'une pompe source (1, 505) et, lorsque la présence d'une pompe source est détectée, réalisation des étapes de :
b) réception, via l'interface de communication (60), d'un programme de perfusion en provenance de la pompe source (1, 505), et
c) sauvegarde du programme de perfusion dans la mémoire (42).

10. Pompe à insuline (1, 505') selon la revendication 9, **caractérisée en ce que** l'unité de commande (40) est configurée pour commander la pompe à insuline (1) après avoir reçu effectivement un programme de perfusion en provenance de la pompe source (1, 505) afin de démarrer la délivrance d'insuline de manière autonome.

11. Pompe à insuline selon soit la revendication 9, soit la revendication 10, **caractérisée en ce que** l'unité de commande (40) est configurée pour détecter un réservoir à insuline (30) de la pompe à insuline (1, 505') en cours de remplissage et, une fois que le réservoir à insuline (30) est rempli, pour activer la recherche d'une pompe source (1, 505).
